Europäisches Patentamt

European Patent Office

Office européen des brevets

(11) Publication number: 0 383 726
A2

# EUROPEAN PATENT APPLICATION

(21) Application number: 90810096.9

(22) Date of filing: 13.02.90

(51) Int. Cl.5: A01H 1/00, C12N 15/00

(30) Priority: 15.02.89 CH 520/89
07.02.90 CH 383/90

(43) Date of publication of application:
22.08.90 Bulletin 90/34

(84) Designated Contracting States:
AT BE CH DE DK ES FR GB GR IT LI LU NL SE

(71) Applicant: SOGEPRE ETABLISSEMENT A
SCHAAN(FL)
Austrasse 42
FL-9490 Vaduz(LI)

(72) Inventor: Bellorini, Luciano, c/o Isabel Meoz
Trans. 12 No. 124-51, Apt. 210
Bogotà(CO)
Inventor: Sassoon, David Randolph
Calle 26 No. 34-14
Bogotà(CO)

(74) Representative: Baggiolini, Raimondo et al
Patent Attorneys
Fiammenghi-Fiammenghi-Racheli Via San
Gottardo 15
CH-6900 Lugano(CH)

(54) A method and apparatus to realize or improve the genetical mutation into seeds and others organic compounds, and to operate photochemical reactions into vegetables and other substances.

(57) The method provides to inject into seeds or material or substances, i.e. to a soya seed, through a laser beam, a plurality of programmable silicon microchips, working as micromotors or micro-machines or microsystems, each of which displace-able and orientable by the energy derived from said laser beam and the aid of a computer with its monitor on which appears the molecular spectrum of each point explored.

The method allows i.e. the production of biodeg-radable resins, of proteins, of pure ammino acids and their derivatives, also from vegetable discards and unvalued materials or substances.

**A method and apparatus to realize or improve the genetical mutation into seeds and others organic compounds, and to operate photochemical reactions into vegetables and other substances.**

The present invention relates to a method and a device to realize or improve the genetical mutation into seeds and other organic compounds, and to operate photochemical reactions into vegetables and other substances, allowing the production of biodegradable resins, of proteins, pure amminoacids and their derivatives, also from vegetable discards and unvalued materials or substances.

Known are methods and apparatuses to realize or improve genetical mutations, employing light of different frequencies and systems.

The method and the apparatus according to the present invention differ from what known in that the sources of energy is given by the laser energy.

More particularly, the method according to the present invention is characterized in that into said seeds, or materials or substances will be injected through laser beams, a plurality of programmable microchips as part of micromotors or micromachines or microsystems having the properties to concentrate its radiation in each point to be explored, each of which, displaceable and orientable by the energy derived from said laser beams with the aid of a computer and a its monitor on which appears the molecular spectrum of each point explored; a servos for remote control from the outside, being employed either to inject, at low temperature, new molecules, or to cut the linkages between molecules, in order to realize or improve the desired genetic mutation and acting at high temperature for destroing molecules if requested; bacterias and or enzimes being also employable, to reach the aim.

The apparatus to realize said method is characterized by a plurality of programmable silicon microchips working as micromotors or micromachines or microsystems, each having the dimensions less than 0,1 micron, to explore the adiacency; the orientation of each micromotor or micromachine or microsystem occuring per piezoelectric effect, through a source of photons delivered by a photocell hitted by the laser beam; a servos for remote control from the outside and a computer with its monitor, on which appears the molecular structure of the points explored by each micromotor or micromachine or microsystem, completing the whole.

The method according to the invention allows therefore to realize or improve the genetical mutation into seed, into the molecular structure of vegetables, or organic compounds, allowing the production of biodegradable resins, of proteins, of pure ammino acids and their derivatives, also from vegetable discards (i.e. from soya and rice skin) and unvalued materials or substances.

Bacterias and or enzimes may be also employed to reach the aim.

The enclosed drawings represent, only in schematical view the principal components of the apparatus according to the present invention.

- Fig. 1 represents, remarkably enlarged, the micromotor or micromachine acting as a microinjector;

- Fig. 2 represents the same micromotor or micromachine acting as a scissor or saw to cut or saw the molecular linkages.

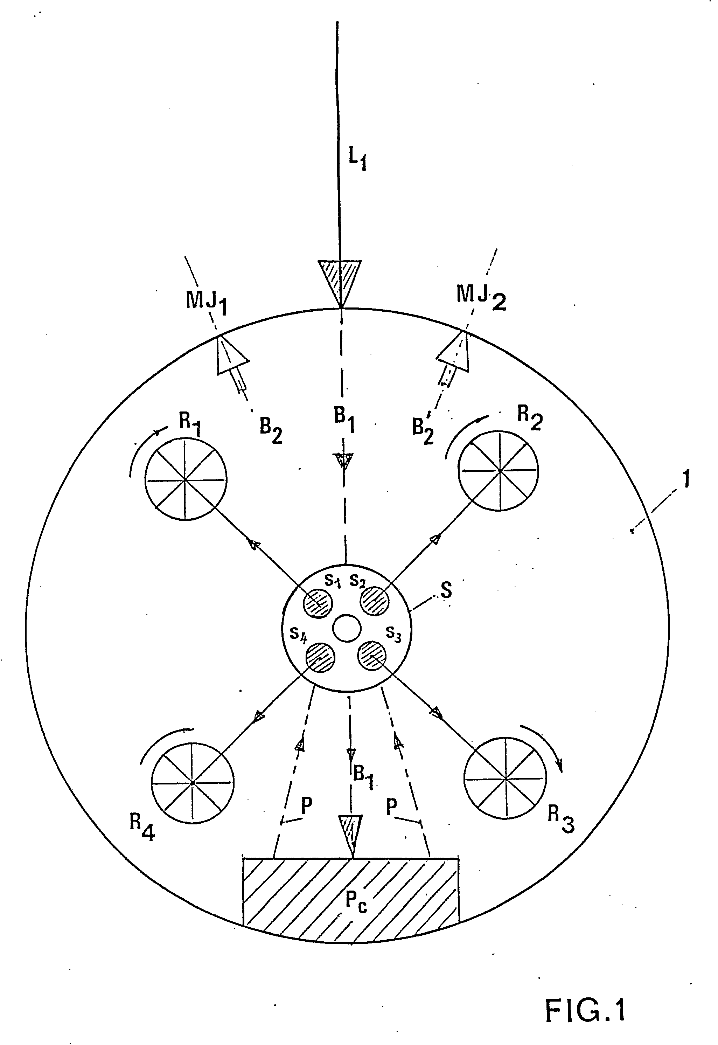

Referring to fig. 1: 1 represents a microchip, $L_1$ a micro-laseriser-apparatus, which beam $B_1$ hitts the photocell or energy cell $P_c$ .The photons P emitted by the photocell $P_c$ give their energy to the servos S for remote control from the outside, servos including the servo-centers $S_1$, $S_2$, $S_3$, $S_4$.

These servo centers give their energy to the rotor R1, $R_2$ , R3, R4, to displace the wohle in the correct position so that the micro-injectors $MJ_1$ , $MJ_2$, by the rays $B_2$, $B'_2$ can operate the injection of molecules in the desidered points of a seed or substance to be subjected to a genetical mutation.

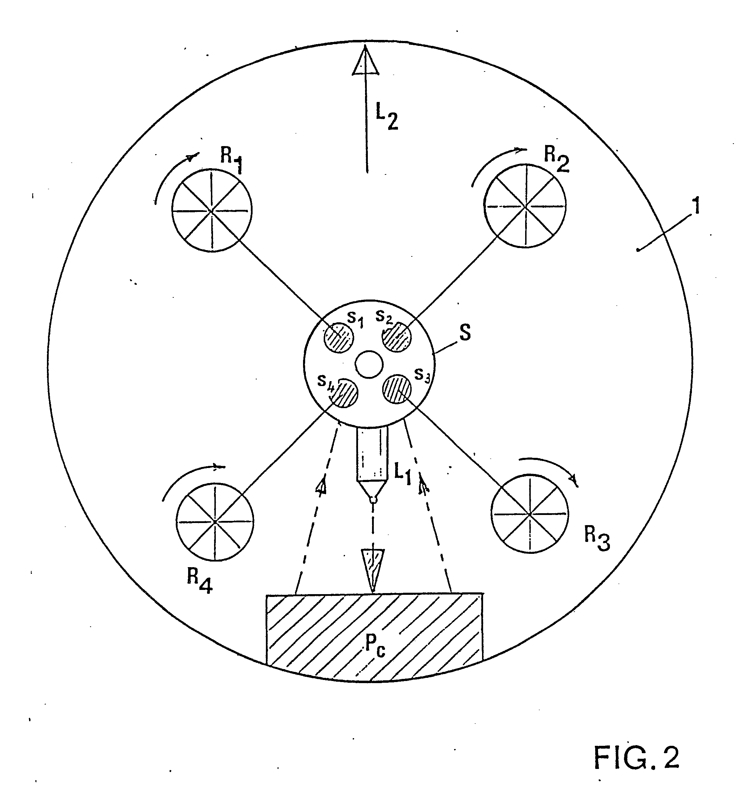

Fig. 2 represents the micromotor or micromachine acting as a scissor or saw to cut or saw the molecular linkages, through the laseriser $L_2$ , schematically indicated by a arrow.

How the micromotor or machine does it work ? How it is made ? Taking a specific microchip 1 and with a new design, by a laser carving the chip and positioning each and every part on it.

Some parts are welded also with laser energy. All of this work is done with the aid of electronic known macroscopes and bio-computers to follow not only the procedures of fabrication but also the process of mutation and microinjection itself.

The system (micromotor or micromachine as we call it) or "T.m.m" works as follows: The L1 (fig. 1) microlaseriser sends an infrared energy-spectrum over the photo-cell Pc or lite cell or energy-cell to produce energy transformed in pulses which will go to the servo center S (fig. 1) and activate the center $S_1$, $S_2$, $S_3$, $S_4$. From there the complete complex mini-system which is activated from the outside through computer, monitor and with the help of servos with initiate the command of the rotors $R_1$, $R_2$ , $R_3$, $R_4$. They move the micro-machine which will go wherever we wanted to go.

This system is a bio-carrier and will deposit in the place needed an array of genetic molecules, in order to change the genetics of a seed (i.e. of soy seeds). Once it arrives the outside post commands the m.m and the micro injector MJ₁, or MJ₂ will inject its bio-cargo.

Sometimes we will put sensors to follow reactions or if not, the m.m. will be discarded as it does not poses any problem due to its size.

To cut or saw the molecular linkages (fig. 2) everything is the same, (see fig. 2) the only difference is that this system carries a lite-cutter in form of a laseriser L₂.

This m.m. will be also useful in micro surgery doing tasks very difficult to perform before.

It is injected in the blood-steam where it goes to a desired place and then it can cut with light power any nerve, any cell, and help in angioplast etc.

It will also solve to monitor other complex problems in the body.

Always according to the present invention, it is provided that:

a) the system be composed of on array of different lasers forming a "screen" interacting various coordinates thus inducing and monitoring ultrafast bio-chemical reactions in order to energize, analize and photo-mutate-genetically products for the agro-industry, for the lab-field research etc.;

b) the system comprises:

a) molecular lasers;

b) vibrational transition gas as a vast mixture of gases depending on the reactions wanted;

c) inert and halides (ions) mixex to react;

d) free electrons which can give energy with the aid of an electromagnetic field.

It could therefore give following definition of the action of the process according to the present invention:

"Photo-bio-chemistry, induced by multilaser radiative energy, tunable, (both in energy units and in changed controlled temperatures)".

In this the present invention differs from what is known, i.e.: "in the laser induced molecular fluorescence" and in the "X_L S_B Raman-spectroscopy".

## Claims

1. A method to realize or improve the genetical mutation into seeds, and other organic compounds, and to operate photochemical reactions into vegetables and other substances, allowing the production of biodegradable resins, of proteins, of pure amminoacids and their derivatives, also from vegetable discards and unvalued materials or substances, characterized in that into said seeds, or materials or substances will be injected through laser beams, a plurality of programmable microchips, as part of micromotors or micromachines or microsystems, having the properties to concentrate a radiations in each point to be explored, each of which displaceable and orientable by the energy derived from said laser beams, with the aid of a computer and a monitor on which appears the molecular spectrum of each point explored; a servos for remote control from the outside being employed either to inject at low temperature, new molecules, or to cut the linkages between molecules in order to realize or improve the desired genetic mutation and acting at higher temperature for destroing molecules if requested; bacterias and or enzimes being also employable, if requested, to reach the aim.

2. The method according to claim 1, applied to improve the qualities of seeds of soya beans, to obtain, after sowing, improved harvestings.

3. Apparatus to realize the method according to claim 1, characterized by a pluralitiy of programmable silicon microchips working as micromotors, or micromachines or microsystems, each having the dimensions less than 0,1 micron, to explore the adiacency; the orientation of each micromotor, or micromachine or microsystem occuring per piezoelectric effect through a source of photons delivered by a photocell hitted by the laser beam; a servos for remote control from the outside and a computer with its monitor on which appears the molecular structure of the points explored by each micromotor, or micromachine or microsystem, completing the whole.

4. A system embodying the method according to claims 1, 2 and the apparatus according to claim 3 characterized in that it comprises an array of different lasers, forming a screen interacting various coordinates, thus inducing and monitoring ultrafast bio-chemical reactions, in order to energize, analize and photo-mutate-genetically, products for the agro-industry, for lab-fields, research and so on. 5. A system embodying the method according to claims 1, 2 and the apparatus according to claim 3, characterized in that it comprises:

a) molecular lasers;

b) vibrational transition gas as a vaste mixture of gases, depending on the reactions wanted;

c) insert halides (ions) mixed to react;

d) free electrons, which can give energy with the aid of an electromagnetic field.

EP 0 383 726 A2

FIG.1

FIG. 2